Europäisches Patentamt

European Patent Office

Office européen des brevets

(11) Publication number: **0 033 222**
**B2**

# NEW EUROPEAN PATENT SPECIFICATION

(45) Date of publication of the new patent specification:
08.06.88

(51) Int. Cl.⁴: **A 61 M 1/14**

(21) Application number: **81300273.0**

(22) Date of filing: **21.01.81**

(54) Dialysate supply circuit.

(30) Priority: **23.01.80 US 114776**

(43) Date of publication of application:
**05.08.81 Bulletin 81/31**

(45) Publication of the grant of the patent:
**28.11.84 Bulletin 84/48**

(45) Mention of the opposition decision:
**08.06.88 Bulletin 88/23**

(84) Designated Contracting States:
**BE CH DE FR GB IT LI NL SE**

(56) References cited:
**BE-A-879 812**
**CH-A-637 019**
**DE-A-2 634 238**
**DE-A-2 838 414**
**DE-C-2 944 136**
**FR-A-2 434 624**
**FR-A-2 440 741**
**JP-A-78 142 382**
**NL-A-7 908 045**
**SE-A-7 909 117**
**US-A-2 034 601**
**US-A-4 096 059**
**US-A-4 209 391**

**Journal of Artificial Organs, Vol.2 (1978), pages 144-146**

(73) Proprietor: **CD Medical, Inc., 999 Brickell Avenue,
Miami Florida 33131 (US)**

(72) Inventor: **Papanek, Thomas H., 6887 Saroni Drive,
Oakland California 94611 (US)**
Inventor: **Egler, Mark S., 445 Marlboro St.Apt.1,
Boston Massachusetts 02115 (US)**
Inventor: **Landau, Julian I., 3769 Northridge Drive,
Concord California 94518 (US)**

(74) Representative: **McCall, John Douglas, W.P.
THOMPSON & CO. Coopers Building Church
Street, Liverpool L1 3AB (GB)**

EP 0 033 222 B2

## Description

This invention relates to an improved single pass dialysate supply apparatus employing a pair of cylinders equipped with hydraulically driven diaphragms, and to a method for controlling the diaphragms and resultant dialysate flow to and from a hemodialyzer during hemodialysis. The apparatus of this invention is of the general type disclosed in U.S. Patent No. 4,029,391 entitled Apparatus and Method for Automatically Controlling Hemodialysis at a Pre-selected Ultrafiltration Rate. The U.S. patent No. 4,209,391 was published after the claimed priority date of the present application and does not form part of the state of the art. It is incorporated into this description and discussed herein to explain the evolution of the present invention as it progressed from such U.S. Patents as 4,021,341 and 4,096,059, mentioned below, to the structure described in this application. The apparatus of this invention provides new means for providing flow of identical liquid volumes of dialysate to and from the kidney so as to attain the same general method of hemodialysis treatment that is achieved by U.S. Patent 4,209,391; the new means are modified and improved relative to the power driven pistons or diaphragms employed in that apparatus; the new means are also simpler and cheaper to manufacture and operate than the means of the prior disclosed apparatus.

Heretofore, systems commonly referred to as closed systems, as in U.S. Patents 4,021,341 and 4,096,059 made the assumption that the volume of spent dialysate supplied to the kidney represents water extracted from the blood in the kidney. This assumption has proved to be incorrect and systems relying on measurements of such excess fluid volume as the ultrafiltrate control mechanism have proved to be inaccurate and commercially unsatisfactory. The source of error in the equal volume assumption employed in connection with prior art closed system was identified in U.S. Patent 4,209,391 and JP-A-142 382/78 and means were provided in its new circuit which solved the problem and eliminated the error in the false assumption by permitting only bubble-free dialysate to enter each of the two cylinders. As the result, the new circuit or U.S. Patent 4,209,391 enabled control of ultrafiltrate rate merely by pre-setting the rate of bubble-free liquid withdrawn from the circuit through the use of an independently controlled ultrafiltrate withdrawal means such as a third piston or a volumetrically controllable pump.

The apparatus of this invention employs hydraulically driven diaphragms in lieu of the power driven pistons or diaphragms; the circuit of this invention is otherwise generally similar to that of U.S. Patent 4,209,391. Early attempts to merely substitute hydraulic drives for positive mechanical drive in the diaphragm embodiment of that circuit were failures. It was found that flexible diaphragms passively driven in response to higher fluid pressure on one side of the diaphragm than on the opposite side faced an additional control problem due to fluctuations or variations in rate of traverse within the cylinder that were not encountered with positively driven diaphragms. It was observed that traverse rate fluctuations were variable with time and uncontrollable because the hydraulic driving pressure on the infeed dialysate was either the dialysate mixing pump pressure or water source line pressure and each such pressure is subject to unexpected variations; in contrast, the driving force in the spent dialysate line of the circuit is mechanically induced hydraulic pressure which results from pump operation that is likewise subject to sporadic variation. Summarily stated, it was found that the hydraulic driving force on the diaphragms in each of the two cylinder units for an entire cylinder length traverse was rarely, if ever, identical. These driving force variations cause the passively driven diaphragms in each of the cylinders to arrive at the end of stroke at different instants in time, with the absolute time difference varying as a function of the pressure variations during a given traverse from one side of the cylinder to the other. Whereas positive drive common to the two cylinders insured simultaneous attainment of end of stroke of the diaphragms in the fresh/drain and kidney/spent circuits, it became necessary with the hydraulically driven diaphragms to elect to switch valves at the instant of end of stroke of either the first or the second diaphragm. Attempts to switch at the instant of the first to arrive at end of stroke failed because of progressive shortening of each stroke; this procedure culminated in more and more rapid switching from cycle to cycle until final arrival in a flutter lock-up condition which is unsatisfactory. Switching at the instant of the second, or latest, end of stroke of the diaphragms in the two cylinders in the separate flow paths produces a no-flow condition of fluid in the circuit of the first to arrive diaphragm during the interim until the second diaphragm achieves its end of stroke.

An arrangement is described in German OLS 2 634 238 in which the system is arranged so as to ensure that the end of stroke of the diaphragm in the infeed flow path always occurs before the end of stroke in the dialysing circuit and that switching occurs at the end of stroke in the dialysis circuit.

It was found however that when the no-flow condition occurred in the infeed, fresh dialysate supply line, undesirable effects resulted in the dialysate make-up portion of the single pass dialysate circuit which supplies the bubble-free fresh dialysate to the circuit of this invention. Alternatively, when the no-flow condition occurs in the spent dialysate removal line that flow of dialysate in the dialyzer is interrupted and diffusion rates across the semipermeable membrane are detrimentally affected. Based upon the undesirable aspects of these options it was found to be necessary to control the traverse of the diaphragms in each of the two cylinders such that the instant of end of stroke in each is synchronized.

The apparatus of this invention provides modified circuit elements and associated control means which achieve the necessary control of diaphragms traverse to attain the needed synchronization to enable successful commercial use of hydraulically driven diaphragms in hemodialysis treatments.

Furthermore, in accordance with the present invention, it is unnecessary to control or preset the transmembrane pressure in the kidney with a hemodialyzer having constant filtration characteristics. This invention makes no attempt to control pressure in the system and relies upon fluid withdrawal to produce inherent pressure conditions to generate from the blood the quantity of water that equals the withdrawn quantity of dialysate; this invention maintains the hydraulic integrity of the entire circuit by insuring that the precise quantity of bubble- free dialysate fed to the kidney does equal the quantity of bubble-free dialysate that is sent to drain, and withdraws only bubble-free ultrafiltrate. Moreover, and as above mentioned to avoid dialysate make-up undesirable effects or irregular flow rates through the kidney, this invention monitors fluid flow rates in the fresh/ drain and the kidney/spent circuits and employs the difference in time of arrival of each of the diaphragms in the two cylinders at its end of stroke to generate signals that in turn are used to change the rate of dialysate flow in the spent dialysate removal line to thereby synchronize the end of stroke instant for the two diaphragms.

Another aspect of the present invention has been the discovery that the hydraulically driven diaphragms in the circuit of this invention must be synchronized in such a manner so as to complete their traverse in each cylinder in each half cycle in the same direction of movement at the same instant in time. "Half-cycle" hereinafter and in the claims refers to traverse of the diaphragm between the ends, or sides, of a cylinder in a single direction. Such synchronization overcomes and offsets or compensates for any variation in driving force on each diaphragm in its cylinder. Each diaphragm functions alternately in the fresh/drain or kidney/spent circuit such that at any given instant in time each diaphragm is monitoring the flow rate in a circuit isolated from the circuit of the other diaphragm. Due to this circuit isolation it is unnecessary that the time interval for each half-cycle be identical; it is also unnecessary that the cylinder volumes be identical. It is, however, desirable that the flow rate during one half- cycle be substantially the same as the flow rate during the other half-cycle. Thus, it is desirable that the cylinder volumes be substantially the same, for example, within ± 5 %.

The new apparatus is modified to include flow measuring means, or switches, in the infeed, fresh bubble-free dialysate line and also in the spent dialysate removal line. These flow switches detect end of stroke of the diaphragms in each circuit and feed an electrical signal to a microprocessor which feeds corrective signals to the pump in the spent dialysate removal line on the following, or next delayed, half-cycle to correct flow rate of the spent dialysate so as to bring about synchronization of the instant of end of stroke in each diaphragm in each half-cycle on a continuing, or continuous, basis. The system also includes a pressure transducer in the spent dialysate removal line, as shown, which detects pressure changes in the spent dialysate caused by patient blood pressure, kidney permeability changes, operator change of ultrafiltrate withdrawal rate, etc., and feeds signals to the microprocessor which, in turn, feeds corrective signals to the pump in the spent dialysate line to restore the rate of flow to its rate prior to any change due to the detected pressure change.

In the accompanying drawings;

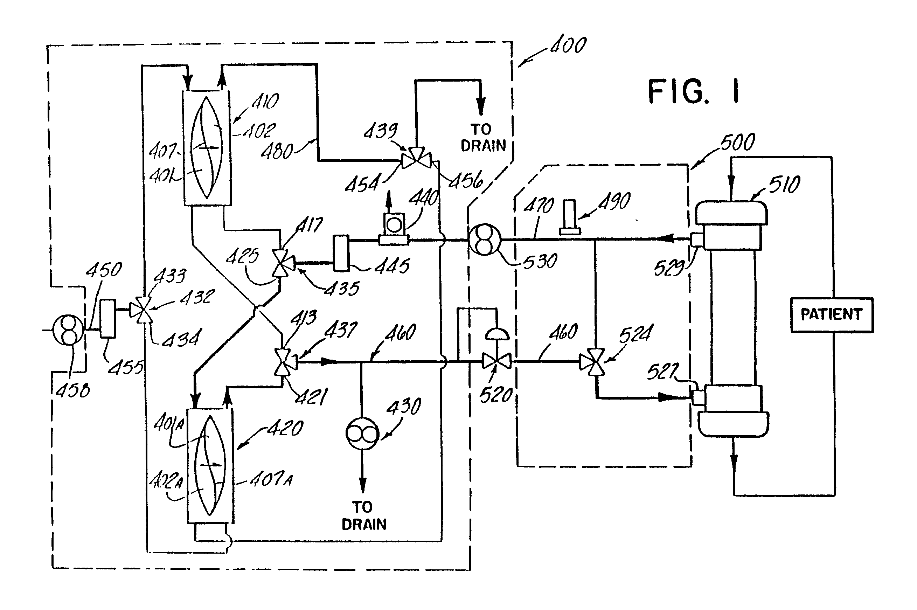

Fig. 1 is a schematic illustration of the improved circuit of this invention showing the dialysate flow path of each cylinder when the switching valves are in valve state A in heavy lines;

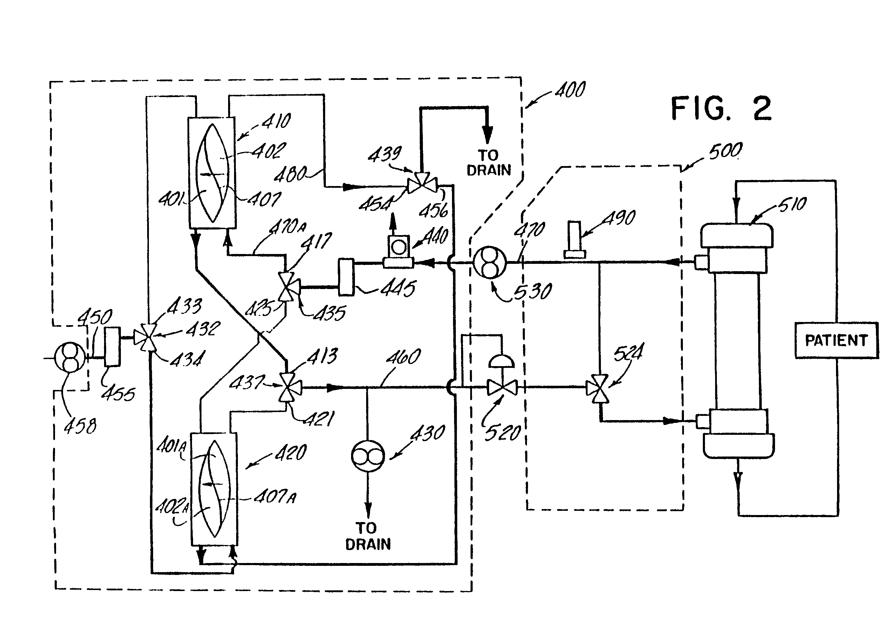

Fig. 2 is a schematic illustration similar to Fig. 1 showing, in heavy lines, the dialysate flow path of each cylinder when the switching valves are in valve state B;

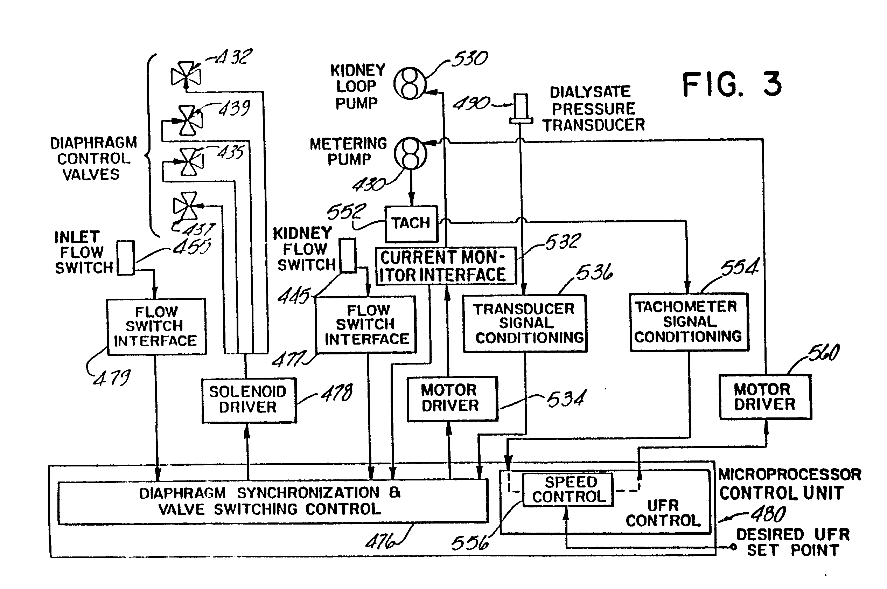

Fig. 3 is a block diagram representation of the interface connections between the operating elements of the circuit as shown in Figs. 1 and 2 and the microprocessor control unit;

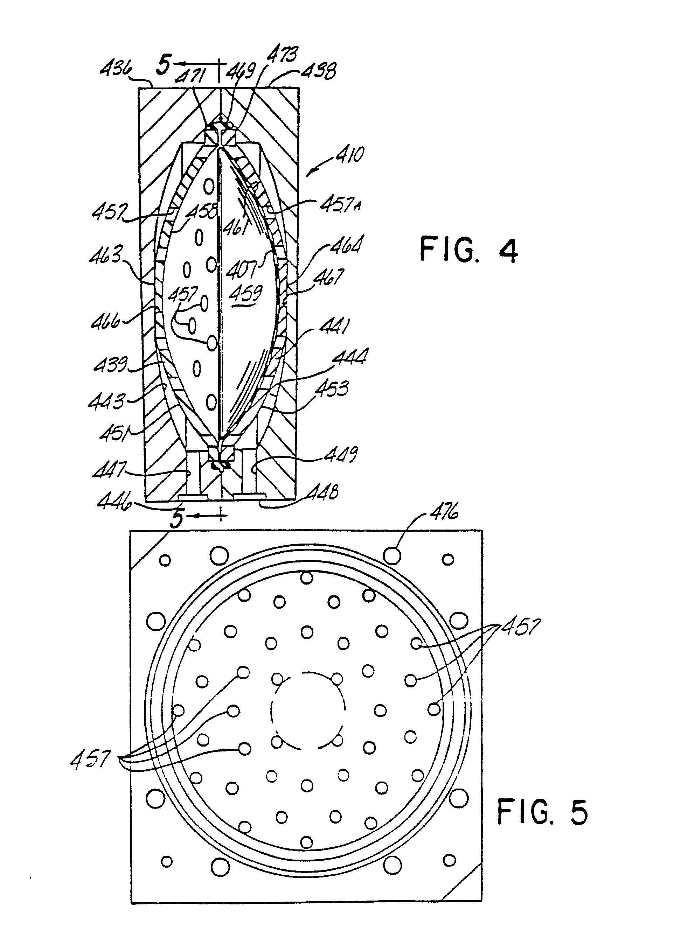

Fig. 4 is a vertical cross-section of one of the cylinder assemblies used in the circuit of this invention showing the hydraulically driven diaphragm in its end of stroke position on the right hand side of the cylinder;

Fig. 5 is an elevation view of a disassembled half of the cylinder assembly of Fig. 4 looking toward the left half and showing the distribution of the dialysate flow path openings in the diaphragm support member;

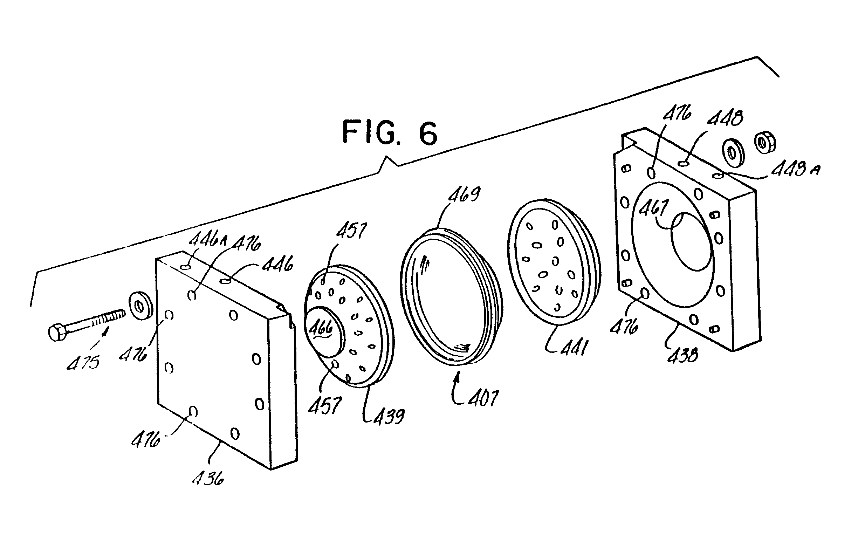

Fig. 6 is an exploded view of the disassembled parts of the cylinder assembly of Figs. 4 and 5;

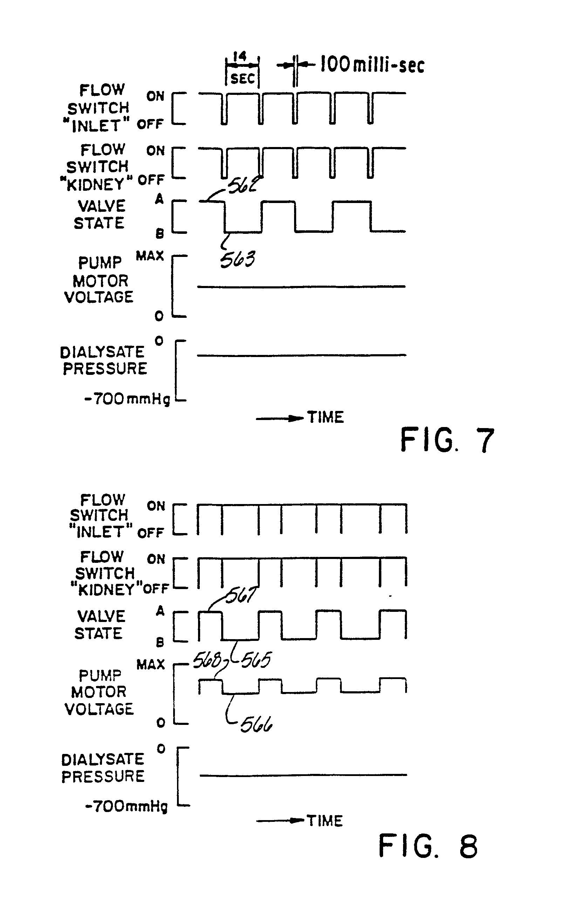

Fig. 7 is the time sequence diagram illustrating the relationship of the flow switches in the inlet, or fresh/drain loop, and in the kidney, or kidney/spent loop, to the pump motor voltage and to dialysate pressure as those switches reflect end of stroke in valve state A (Fig. 1) and valve state B (Fig. 2), and specifically illustrating steady state operation when the diaphragms in the two cylinders are in synchronization for both valve states A and B;

Fig. 8 is a time sequence diagram of the type shown in Fig. 7 which illustrates the operating condition of greater resistance to flow in the fresh/drain circuit in valve state B than in valve state A and specifically showing synchronized end of strokes of both diaphragms where the half-cycle time in valve state B exceeds that in valve state A;

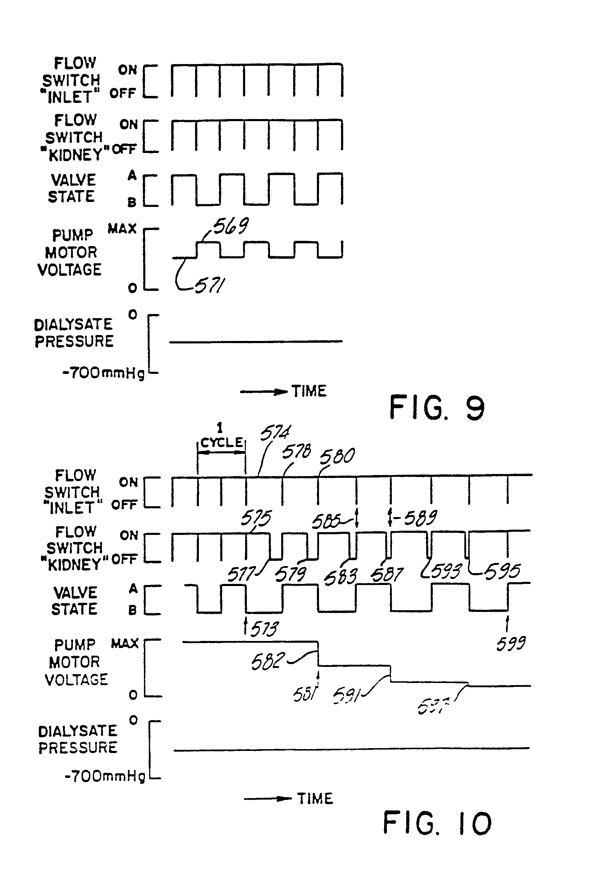

Fig. 9 is a time sequence diagram of the type shown in Fig. 7 which illustrates the operating condition of greater resistance to flow in the kidney/spent circuit in valve state B than in valve state A and equal resistance to flow in both valve states A and B in the fresh/drain circuit and specifically showing synchronized end of strokes of both diaphragms in equal halfcycle time periods resulting from the use of increased voltage to the pump means in the kidney/spent circuit during valve state B;

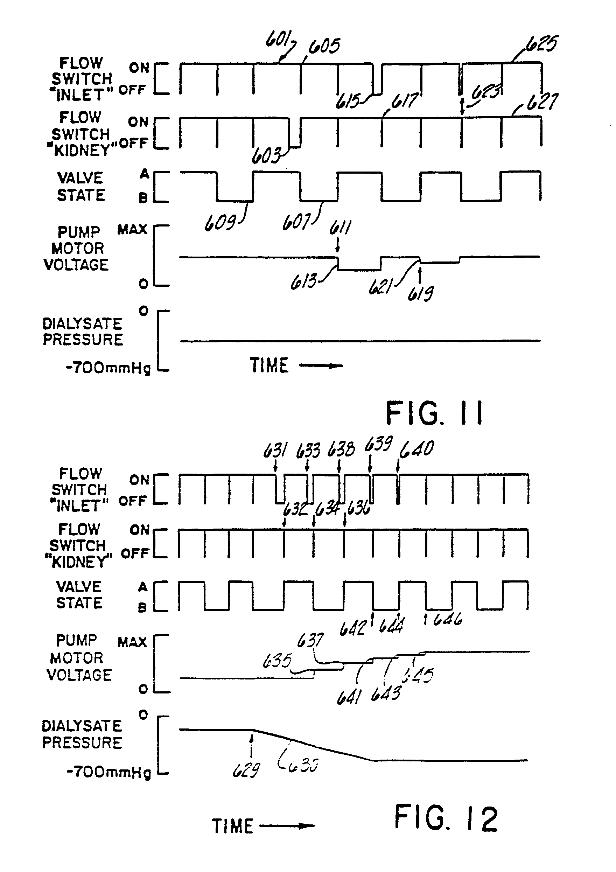

Fig. 10 is a time sequence diagram of the type shown in Fig. 7 which illustrates the operating condition resulting from a shift in incoming dialysate pressure in the fresh/drain circuit to a new lower level having a similar effect on both circuits in valve states A and B specifically illustrating the half-cycle delayed adjustments made by the microprocessor to cause the kidney/spent circuit diaphragms to traverse their cylinders in the same elapsed time as the lengthened time required in the inlet or fresh/drain circuit such that the diaphragms become synchronized;

Fig. 11 is a time sequence diagram of the type shown in Fig. 7 which illustrates the operating conditions resulting from a momentary, single half-cycle reduced flow rate in the fresh/drain circuit in valve state A, only, and illustrating microprocessor corrections in succeeding, delayed half-cycles to re-establish diaphragm synchronization;

Fig. 12 is a time sequence diagram of the type shown in Fig. 7 which illustrates the operating condition resulting from an operator setting of high ultrafiltrate withdrawal rate and specifically illustrating the successive changes in pump motor voltage to achieve synchronization at a numerically greater negative pressure across the semi-permeable membrane through detected differences in end of stroke of the diaphragms.

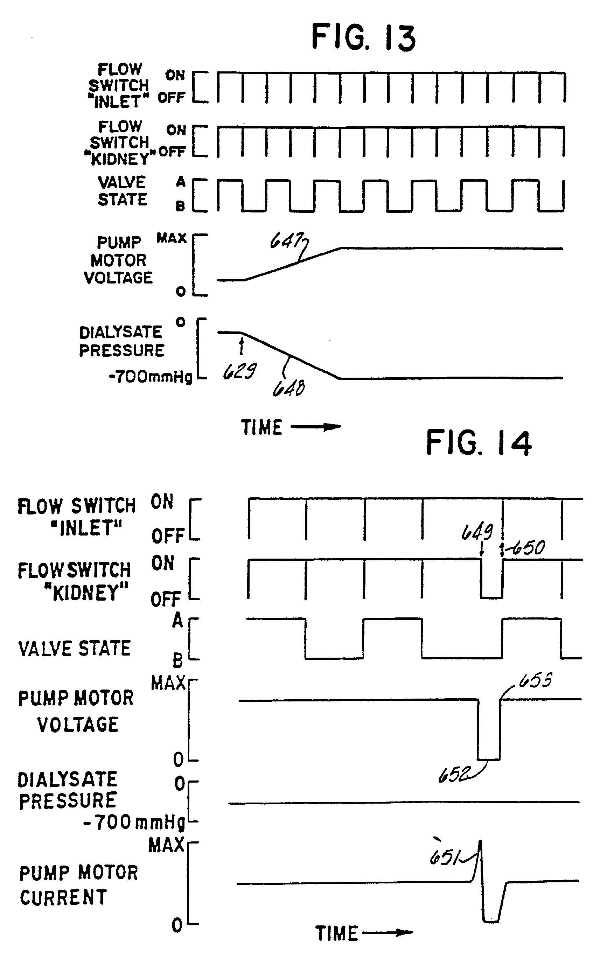

Fig. 13 is a time sequence diagram of the type shown in Fig. 12 and illustrating the effect of optional inclusion in the circuit of a pressure transducer which continuously monitors dialysate pressure changes in the low pressure lines and changes pump voltage to compensate for pressure-induced changes in pump output independently of voltage changes dictated by detected differences in the instant of the end of stroke of the two diaphragms.

Fig. 14 is a time sequence diagram of the type shown in Fig. 7 which illustrates an embodiment of the invention insuring safe operation of the circuit during the period of no flow of the fluid in the spent dialysate removal line due to diaphragm arrival at end of stroke in that line prior to arrival at end of stroke of the diaphragm in the fresh/drain circuit in either valve state A or B.

As may be seen in the Figures 1 and 2 the circuit of this invention comprises an above atmospheric pressure portion which includes those elements circumscribed by the dash line generally designated 400 and a below atmospheric pressure portion containing the elements enclosed by the dash line area generally designated 500. The functional components, or elements, of the circuit which remain at all times above atmospheric pressure include cylinder units 410 and 420, upstream fresh dialysate flow-inducing means 458, inlet dialysate flow detecting means 455, and valves 432, 435, 437 and 439 as shown in Fig. 1 and Fig. 2, ultrafiltrate removal means or metering pump 430, degasifier 440 and spent dialysate removal line flow detecting means, or switch, 445. The components in the below atmospheric pressure portion 500 include the dialysate chamber portion of the hemodialyzer generally designated 510 and the optionally present dialysate detector means 490. The main components between the above atmospheric pressure portion 400 and the below atmospheric pressure portion 500 are the pressure reducing means generally designated 520, and spent dialysate flow generating, or pump, means generally designated 530. Spent dialysate pressure detector means 490 when optionally present satisfactorily is located in either the fresh dialysate line 460 or preferably is located in the spent dialysate line 470, as shown. Hemodialyzer 510 may be any of the commercial types but preferably the semipermeable membranes are hollow fibers.

The above identified functional and main components in the above and below atmospheric pressure portions, respectively, are interconnected in the closed hydraulic circuits shown in Fig. 1 which corresponds to valve state A, and in Fig. 2, which corresponds to valve state B, on an alternating time basis during use. Taken collectively the circuits of Figs. 1 and 2 provide a continuous supply of fresh dialysate to and remove spent dialysate from artificial kidney 510 and enables a set ultrafiltrate withdrawal rate to cause the withdrawal of bubble- free ultrafiltrate, or water, to be extracted from the blood; the set ultrafiltrate withdrawal rate constitutes the control parameter which instantaneously and continuously induces pressure changes between the blood flowing on one side of the semipermeable membrane in the kidney and the dialysate flowing on the other side of that membrane to cause the desired quantity of water to be so removed at the desired rate of such removal. This mode of operation is generally similar to that of U.S. Patent 4,209,391, but differs therefrom in specifics and on an instantaneous basis consequent from the use in the dialysate cylinders 410 and 420 of passively driven diaphragms as opposed to power driven pistons or diaphragms.

The functional relationships between the elements in the circuits as interconnected during alternating half-cycles of dialysate flow will, for simplicity, be explained with specific reference to the state of the valve generally designated 432 in incoming fresh dialysate supply line 450 under the designations valve state A and valve state B. Valve state A is illustrated in Fig. 1 wherein dialysate flow is indicated by the heavy lines in the directions of the arrows thereon. Valve state B is illustrated in a similar manner in Fig. 2.

During valve state A, Fig. 1, port 433 is open and port 434 is closed; during this half-cycle fresh dialysate flows through supply line 450, through flow detecting means 455, through open port 433 and line 435 into chamber 401 of cylinder 410. Fresh dialysate is supplied through line 450 in premixed bubble-free form and typically at 37°C and under a positive pressure of about 13,8 to about 68,9 Pascals (about 2 to about 10 pounds per square inch). The positive pressure at valve 432 provides the propelling force for the dialysate in the fresh/ drain circuit and may be produced, or controlled, by pump 458, or equivalent means such as a pressure regulator, not shown, or by other flow inducing means located in an upstream dialysate generating circuit.

In valve state A the inflow of dialysate into chamber 401 causes hydraulically driven diaphragm 407 to move to the right, in the direction of the arrow, and to expel from chamber 402 spent dialysate through line 480 and through port 454 of valve 439 to drain. Thus, during valve state A, cylinder 410 performs the functions of receiving fresh dialysate and sending spent, or effluent, dialysate to drain and the circuit performing these functions will hereinafter be designated the fresh/drain circuit. During this same time elapse in the valve state A half-cycle, fresh dialysate is supplied to kidney 510 from chamber 401A of cylinder 420 and spent dialysate is received from kidney 510 in chamber 402A; it is to be understood that all valves in the circuits, except by-pass valve 524, operate to open or close at substantially the same instant in time such that dialysate flow remains substantially constant to and from kidney 510 at all times, that while cylinder 410 is filling with fresh dialysate, cylinder 420 is filling with spent dialysate and that during the succeeding half-cycle the cylinder functions reverse. The driving force for spent dialysate flow in spent dialysate line 470 is power driven means 530, usually a pump which satisfactorily may be a positive displacement pump, or preferably is a more inexpensive pump such as a gear pump. The control of rate of flow of spent dialysate through detection of the instant of end of stroke of the passive diaphragms in cylinders 410 and 420 is important and will be explained hereinafter in greater detail in connection with the automated control modifications and method of operation. As pump means 530 propels spent dialysate from outlet port 529 in kidney 510 through bubble removal means 440, and flow rate detector 445, through open port 425 of valve 435 into chamber 402A of cylinder 420, passive diaphragms 407A is forced to move to the right, in the direction of the arrow, and to expel the fresh dialysate in chamber 401A, through open port 421 of valve 437 along line 460 through pressure reducing means 520 to the inlet port 527 of kidney 510. Thus during valve state A cylinder 420 performs the functions of sending bubble-free fresh dialysate to the kidney and receiving spent dialysate from the kidney and the circuit performing these functions will hereinafter be designated the kidney/spent circuit. It should be noted that during the valve state. A half-cycle, both hydraulically driven diaphragms 407, 407A move in the same direction and physically reach the right hand end of cylinders 410 and 420 at approximately the same instant in time, with specific variance being a function of varying dialysate rates of flow in the two cylinders, as above generally stated.

Referring now to Fig. 2, the interconnection between the elements in the circuit in valve state B is illustrated. The reversal of functions of cylinders 410 and 420 results from the new interconnections of the circuit elements when ports 433, 454 are closed and ports 434 and 456 are simultaneously opened. By these valve changes fresh dialysate is shunted to cylinder 420; at the same instant valves 437 and 435 are reversed thereby shunting spent dialysate to cylinder 410 through line 470A as fresh dialysate is forwarded to kidney 510 through line 460. By referring to the heavy lines in Fig. 2 it may be seen that cylinder 410 is operatively connected into the kidney/spent circuit while cylinder 420 is connected into the fresh/drain circuit. It may also be seen that both of diaphragms 407, 407A move in the direction of the arrows, toward the left during the valve state B half-cycle and arrive at their end of stroke at the left hand end, or side, of their respective cylinders 410 and 420.

In each of the circuits shown in Fig. 1 and 2 ultrafiltrate withdrawal means generally designated 430 is interconnected between drain and, as shown, fresh dialysate supply line 460 at a location within the above atmospheric pressure portion 400. However, ultrafiltrate withdrawal, or metering, means 430 satisfactorily may be connected between line 470 and drain at any other point between bubble removal means 440 and valve 435.

Dialysate cylinder units 410 and 420 which are schematically shown in Figs. 1 and 2 are similar in construction and are illustrated in greater detail in their preferred form in Figs. 4, 5 and 6. Referring to Fig. 4 and with specific reference to cylinder 410, diaphragm 407 is shown in its end of stroke position on the right hand side of cylinder 410. Cylinder unit 410 is an integrated assembly consisting of two mirror image end plates 436, 438, a first diaphragm support member 439 for assembly into left hand end plate 436, a second diaphragm support member 441 for assembly into right hand end plate 438, and diaphragm 407. End plate 436 is provided with a generally hemispherical shape dished out recess chamber 443 which interconnects with outlet port 446 through channel 447 and with an inlet port 446A. In a similar manner, end plate 438 is provided with a similar shape recess chamber 444 which interconnects with outlet port 448 through channel 449 and an inlet port 448A. Ports 446, 448 as shown in Fig. 4 are shown on the lower end surface of plates 436, 438 but are preferably located on the upper, or top edge surface of end plates 436, 438 when physically installed for use in the circuit to thereby insure avoidance of gas bubble entrapment during start up of operation, or the like. Each chamber 443, 444 is provided with a centrally located circular support surface 463, 464 which receives a mating projection, or boss 466, 467 extending outwardly from the rear surface of each support member 439, 441, respectively to counteract pressure forces on diaphragm 407 at end of stroke.

Each diaphragm support member, or plate, 439, 441 is dish shaped at a selected nonhemispherical curvature adpated to produce upon assembly the desired cavity between the rear wall, surfaces 451, 454 of plates 439, 441, respectively, and the inner surfaces of the chambers 443, 444 in end plates 436, 438, respectively. Each support member 439, 441 is provided with a plurality of radially and circumferentially spaced apertures 457, 457A which extend through the wall of each support member and provide a flow path for dialysate, or other liquid between the central cavity 459 of oblate shape defined by the inner walls 458, 461 of support members 439, 441, respectively and the cavity in communication with it as diaphragm 407 moves from one side of cavity 459 to the other during traverse in alternate valve states A and B. As shown in Fig. 4, diaphragm 407 is in position at the end of stroke of valve state A, Fig. 1, and was forced to the right by dialysate fluid entering chamber 443 through an inlet port 446A, as that dialysate flowed through apertures 457 and exerted pressure on the left hand surface of diaphragm 407 in excess of the fluid pressure on the other side of the diaphragm to thereby cause dialysate flow through apertures 457A into chamber 444 and exit to drain through port 448. It will be seen that diaphragm 407 serves as a closure valve for each aperture 457A in support member 441, and as that flexible diaphragm moves into contact with inner wall 461 all fluid is expelled into chamber 444 due to the distribution of such apertures over the entire surface of inner wall 461. Support members 439, 441 serve the further function of preventing malfunction in full diaphragm traverse and consequent failure to expel in each half cycle all of the dialysate received during the immediately prior half-cycle.

Diaphragm 407 is satisfactorily fabricated from any thin, flexible resilient sheet material such as rubber, or plastic materials and is secured and hydraulically sealed between end plates 436 and 438 by peripheral bead 469 which is wedged between and overlies the peripheral edge of the flanges 471 and 473 of support members 439 and 441, respectively, as shown. As best seen in Fig. 6, cylinder 410 is assembled by first positioning support members 439, 441 in their end plates 436, 438, respectively. Diaphragm 407 is then placed into position and the parts are unified by a plurality of fasteners, such as the illustrated bolt, washer and nut generally designated 475, positioned and tightened in circumferentially spaced apertures 476 in members 439, 441.

An automated method of operation of the circuit will now be described:

Referring to Fig. 3 the overall dialysate flow from cylinders 410 and 420 to and from artificial kidney 510 during valve states A and B, as above generally described, is effected automatically through electronic controls which function to monitor the flow rate of spent dialysate in line 470 and fresh dialysate in inlet line 450; kidney flow rate sensing means 445 in line 470 and inlet flow switch 455 in fresh dialysate supply line 450 detect the instant of end of stroke of diaphragms 407 and 407A in dialysate cylinders 410 and 420, respectively, on a continuous basis. These detected instants of diaphragm end of stroke are relayed to Microprocessor Control Unit, generally designated 480, and particularly to the Diaphragm Synchronization and Valve Switching Control Unit, 476, by electrical signals from Flow Switch Interface Units 477, 479. Control unit 476 is pre-programmed to activate Solenoid Drive 478 to reverse each of the valves 432, 435, 437 and 439 the instant the second of the two diaphragms 407 or 407A arrives at its end of stroke. Detection of diaphragm end of stroke may equally satisfactorily be performed by other means than flow rate sensing means 445 and 455 as will be readily und understood by those skilled in the art. Satisfactorily means include, for example, contact switches, pressure sensing means, optical sensors, fluidic sensing means associated with each cylinder, or the like.

Under perfect, or ideal steady-state, operating condition each of diaphragms 407 and 407A arrive at its end of stroke at the identical instant in time; normally, however, the instant of diaphragm end of stroke is not identical but so rather is milliseconds apart in time. The function of the control circuitry shown in Fig. 3 is to provide corrective electrical signals to means controlling the dialysate flow rate in spent dialysate line 470 to so change that flow rate, on an instantaneous basic, to sychronize the end of stroke of diaphragms 407, 407A in each half cycle of diaphram traverse in cylinders 410, 420.

Kidney loop pump 530 is connected to Diaphragm Synchronization Controll 476 through Motor Driver 534 and optionally present Current Monitor Interface 532 and its rate of operation is increased, or decreased, as required through ampere or voltage control, by Motor Drive 534, as will be more fully described in connection with the time sequence diagrams Figs. 7-14 inclusive. Dialysate Pressure Transducer 490 is interconnected to Diaphragm Synchronization Control 476 through Transducer Signal Conditioning unit 536; signals from transducer 490 are relayed substantially continuously, for example, at 50 millisecond intervals to indicate changes in pressure that effect flow rate through kidney loop pump 530. Diaphragm synchronization control 476, in response to the signal indicating pressure change activates the motor driver 534 to alter the rate of operation of pump 530 to counteract the flow rate change resulting from the pressure change and reestablish the flow rate to the level prior to the change in pressure.

Pump 430 is associated with tachometer 552 which relays the rate of pump 430 operation through Tachometer Signal Conditioning Unit 554 to Speed Control Means 556, a part of Microprocessor Control Unit 480; metering pump 430 is preset by the operator to the desired output rate for ultrafiltrate removal from the above atmospheric portion of the circuit by manual setting of the desired UFR Set Point. This setting is relayed by electrical signal through the Speed Control unit 556 which in turn activates Motor Driver 560, which feeds a control signal, which may be voltage or ampere regulation, to cause metering pump 430 to operate at the speed required to achieve the preset ultrafiltrate flow rate.

The diaphragm synchronization operation will now be described:

Automatic operation of cylinders 410, 420 to alternately connect each with kidney 510 is intended to maintain a substantially constant rate of dialysate flow through the circuit. The objective of the control system is to react to changes in steady-state flow conditions at any point, or points, in the circuit which without correction would change the desired constant flow rate. The method employed is to detect the time difference between the instant of each end of stroke of each diaphragm and this is accomplished in the preferred embodiment of this invention by signals from flow switches 445 and 455 which are fed to control 476. Control 476 senses the incoming signals on a continuous basis and instantaneously converts the time difference into a correcting signal which varies the voltage to motor driver 534, as required, to change the flow rate in line 470 to equal the flow rate in line 450 when the diaphragms are next again moving in the same direction that they were moving to cause the detected time difference between the instant of end of strokes in that half cycle. While detection of time differences and the computation of the corrective voltage change occurs substantially instantaneously, the necessary synchronization of the rates of relative movement of the diaphragms requires a delay in applying that correction until the arrival of the next valve state which is the same valve state in which the difference first occurred, for example, valve state A. Thus, diaphragms synchronization control 476 detects the time difference but delays feeding the correcting signal to motor driver 534 until the beginning instant of the next succeeding half cycle during which flow is again occurring in valve state A. In normal operation traverse of the diaphragms occurs in about 13-15 seconds and a correcting signal applied to kidney pump 530 at the instant the valves switch from valve state B to valve state A has the full half-cycle to remain in effect and bring the flow rate in line 470 to the exact equal of that in line 450 by the end of that half cycle. Ordinarily, however, a single correction does not re-establish exact synchronization due to other changes that may occur at the other points in the circuit during the half cycle in which the correction is being applied and it is necessary to continuously detect time differences in the instants of diaphragm end of stroke, in both half cycles, and to constantly, and repeatedly, convert the detected time difference to corrective signals and to repetitively apply those signals, after delay, to the succeeding next half cycle of diaphragm operation. These principles of operation are illustrated in Figs. 7-14 which show the control circuitry reaction and corrective action taken to overcome various types of flow rate changes which may occur during a hemodialysis treatment of 3 to 5 hours duration.

Figs. 7-14 are time sequence diagrams. The variable time is plotted along the horizontal axis; the instantaneous condition of controlling circuit components and operating parameter levels which result from corrections to pump motor voltage to achieve diaphragm synchronization are plotted along the vertical axis. The diagrams show sequential half cycles in valve state A and valve state B and correlate on an instantaneous basis the status of Inlet Flow Switch 455 and Kidney Flow Switch 445 during each valve state. Flow switches are either "on" or "off" and the horizontal line plotted opposite each position shows the time duration of dialysate flow in that position for each of valve state A or B and the vertical lines indicate the instant of the end of each diaphragm stroke; the exact instant of the change from valve state A to valve state B is shown by the position of the vertical line plotted opposite the legend Valve State A or B.

As may be seen in Fig. 7 the normal time elapse in each of Valve State A and B is 14 seconds and the normal time elapse during the change from one valve state to the other is approximately 100 milli-seconds. As there shown the width of the time elapse during valve switching is exaggerated to illustrate that both flow switches must reach their "off" position before the valves switch from valve state A to valve state B, and vice versa. By using the horizontal line 562 opposite Valve State A or line 563 opposite Valve State B as the control and extending an imaginary vertical line one can observe the instantaneous status of each of the inlet and kidney flow switches, and thus determined the time elapse for each switch to reach its off position. As shown in Fig. 7, the time elapse in Valve State A equals the time elapse in Valve State B and the diaphragm are reaching their end of stroke position at the same instant and are synchronized; the circuit is operating perfectly, and in ideal steady-state condition. In this mode no corrective changes are needed and the pump motor voltage and dialysate pressure remain steady.

In Fig. 8, another type of steady-state operation is illustrated. As may be seen, the rate of dialysate flow in valve state B is slower than during valve state A as indicated by the longer time elapse of line 565 relative to line 567; this reflects greater resistance to flow at some location within the circuit of Fig. 2 in the fresh/drain circuit. The slower rate of flow during valve state B is reflected by lower pump motor voltage as shown in line 566 relative to line 568; the inlet and kidney flow switches nevertheless reach their off positions at the same time instant in both of Valve States A and B and the diaphragms are synchronized because of the corrective signals to the pump 530.

Fig. 9 illustrates a circuit condition in which there is greater resistance to dialysate flow in Valve State B at some location in the kidney/spent circuit than in the kidney/spent circuit in Valve State A. As shown, the pump motor voltage line 569 during Valve State B is higher than the pump motor voltage line 571 during Valve State A to overcome that greater resistance. During valve state B kidney pump 530 is supplied higher voltage sufficient to cause the flow rate in line 470 to equal that in line 450 so that the diaphragms are synchronized and operation is steady-state, as depicted.

The circuit reaction to a drop in dialysate flow rate in inlet line 450 following a prior steady-state period of operation is illustrated on Fig. 10. Such a change could occur due to any external disturbance in the upstream dialysate generation circuit as, for example, a slow down of inlet dialysate pump 458 or the like. As may be seen in Fig. 10, two half-cycles in valve state A and one half cycle in valve state B were completed with the diaphragms in synchronization; a disturbance in the fresh/drain circuit occurred at the beginning of the half cycle in valve state B indicated at 573, and the inlet flow switch remained in its "on" position longer than in previous half-cycles which indicates an increased time of diaphragm traverse. This may be seen by comparing line 574 with kidney switch line 575. Kidney flow switch 445 signaled end of stroke of diaphragm 407 in cylinder 410 at its left hand extremity, Fig. 2, as indicated at 577 while diaphragm 407A remained in traverse until it reached its left hand extremity end of stroke as indicated at 578, at which time the valves switched to valve state A. A similar effect on diaphragm synchronization also occurred in the subsequent half-cycle Valve State A due to the inlet line flow rate decrease, and again kidney flow switch 445 signalled end of stroke of diaphragm 407A before diaphragm 407, Fig. 1, as may be seen by comparing points 579 and 580. These time differences are detected by control 476 and converted into corrective electrical signals for actuating motor driver 534. The first corrective signal, to correct the detected time differences in valve state B at instant 578 is applied one half-cycle later at the instant generally designated 581 which is the beginning of valve state B operation. The correction is an electrical signal which reduces pump motor voltage as shown by line 582. This correction reduced the time difference in valve state B by reducing the flow rate in line 470 thus causing kidney switch 445 to delay its operation at 583 relative to actuation of inlet switch 455 at instant 585. With the pump motor voltage remaining at its new lower level throughout valve state A, a similar reduction occurs in the actuation of kidney switch 445 at 587 relative to actuation of inlet switch at instant 589. A second reduction in pump motor voltage is applied at instant 589 as indicated at line 591, and during the succeeding half-cycles of valve state B and valve state A, a further decrease in the difference between the instants of ends of stroke occurred as shown at 593 and 595, and after a third correction of voltage at instant 595 as shown at line 597, synchronization was attained at instant 599 which is at the end of the third half-cycle in valve state B after the disturbance was first detected at instant 578.

Fig. 11 illustrates a momentary reduction in dialysate flow rate in the fresh/drain circuit during valve state A which lasts for less than a complete half-cycle and does not continue into valve state B. As may be seen in Fig. 11 the reduced flow rate in valve state A occurs at instant 601 causing an earlier actuation of kidney switch 445 at instant 603 relative to actuation of inlet switch 455 at instant 605. The disturbance does not alter the flow rates in valve state B and the time elapse shown by line 607 following the disturbance is the same as the time elapse shown by line 609 prior to the disturbance. At instant 611, one half-cycle delayed after the time difference was detected at instant 605, a correction reducing pump motor voltage was made in the amount indicated by line 613. The correction caused too large a reduction in flow in line 470 during the corrected half-cycle in valve state A, as reflected by the early actuation of flow switch 455 at instant 615 prior to kidney switch 445 at instant 617. This over-correction was off-set on the succeeding half-cycle in valve state A at instant 619 by a smaller voltage reduction as indicated by line 621, and this correction reduced the amount of early actuation of inlet switch 455 relative to kidney flow switch 445 as indicated at instant 623, and thereafter synchronization was achieved in the succeeding valve state A half-cycle as may be seen by comparing line 625 and line 627.

In Figs. 12 and 13 the effect of operator change during the hemodialysis treatment to increase the rate of ultrafiltrate withdrawal is illustrated. Fig. 12 shows automatic adjustment to counteract the change in UFR withdrawal rate when the circuit employs the preferred gear pump 530, or its equivalent, and does not include the optionally present dialysate pressure transducer 490. Fig. 13 illustrates control circuit operation to the same change in UFR withdrawal rate in the same circuit except that pressure transducer 490 is present in the circuit. As shown in Figs. 12 and 13, the operator increases the UFR withdrawal rate at instant 629; to produce the demanded increase in ultrafiltrate the closed, hydraulic tight, bubble-free dialysate flow circuit causes the dialysate pressure to decrease, gradually as indicated by the uniformly sloping line 630. In the valve state B half-cycle after the UFR increase the inlet flow switch closes at instant 631 earlier than the kidney flow switch at instant 632, and in the succeeding valve state A half-cycle inlet switch 455 again closes early at instant 633 relative to kidney flow switch 445 as the dialysate pressure continues to gradually decrease. At the beginning of the next valve state B half-cycle after the early closure at instant 631 an upward voltage correction 635 is made at instant 634 to increase the speed of kidney pump 530 as an offset to the effect that the dialysate pressure drop has on the gear pump output. A similar upward correction in pump motor voltage is made at instant 636 at the beginning of the valve state A half-cycle as indicated at 637 to correct the early close at instant 633. The gradual dialysate pressure decrease continues to cause early closures of the inlet flow switch 455 at instant 638 in valve state B, at instant 639 in valve state A and at instant 640 in valve state B. An upward pump motor voltage correction 641 is made at instant 642 to offset the early closure at instant 638; another upward correction 643 is made at instant 644 at the beginning of valve state A to offset the early closure at instant 639. Another correction 645 is made at instant 646 to offset the early closure in valve state B at instant 640 and thereafter the diaphragms are in synchronization and operating in steady-state at the increased ultrafiltrate withdrawal rate set 84 seconds earlier at instant 629.

In contrast to the five successive corrections to pump motor voltage to again attain synchronization as shown in Fig. 12, the presence of pressure transducer 490 in the circuit assures continuation of synchronization during all pressure changes in kidney 510. Pressure transducer 490 functions by monitoring pressure changes in spent dialysate line 470 at 50 milli-second intervals and sends an electrical signal generated by Transducer Signal Conditioning Unit 536 to Diaphragm Synchronization Control 476. Control 476, pre- programmed to initiate kidney pump voltage corrections to offset such pressure changes, signals through Motor Driver 534 a small voltage increase to cause kidney pump 530 to speed up slightly and restore the flow rate through pump 530 to that existing prior to the pressure change detected by pressure transducer 490. Successive 50 milli-second spaced monitoring signals produce similar small corrections. As shown in Fig. 13 pump motor voltage is gradually and smoothly increased along line 647 after the ultrafiltrate withdrawal rate was increased at instant 629 and dialysate pressure gradually reached the same lower pressure level along line 648 that was reached in the operation of the circuit without transducer 490 as shown in Fig, 12. Throughout the small voltage corrections in Fig. 13 the diaphragms remained in synchronization and all voltage corrections were completed in four half-cycles or in 56 seconds and terminated substantially at the same instant the new lower pressure was reached in kidney 510.

Fig. 14 illustrates an embodiment of the invention which is operative when optionally present Current Monitor Interface 532 is present in the circuit. Current Monitor Interface 532 monitors the slope of the line determined by current as a function of time. Interface 532 is useful in a circuit that employs a pump driven by a direct current permanent magnet motor. In such motors the current draw is proportional to output load and in the event flow becomes blocked in the output line the current rises sharply. Such output line blockage does occur each time the diaphragm in the kidney/spent circuit reaches its end of stroke before the diaphragm in the fresh/drain circuit arrives at its end of stroke. In that interim, pump 530 continues to receive its normal operating voltage and continues to pump toward the diaphragm in the now filled kidney/spent cylinder such that pressure rapidly increases in the circuit elements between the outlet of pump 530 and the outlet of the cylinder in the kidney/spent circuit which includes bubble trap 440, flow switch 445 and the valves. Such pressure increases may cause rupture of valve seals, or puncture the diaphragm or disable the exit vent on trap 440 and should therefore be avoided. In Fig. 14, the automatic control circuit response to such an event is shown. After three half- cycles of steady-state operation an event occurs such as a momentary reduction in the flow in the fresh/drain circuit which causes the diaphragm in the kidney/spent line to arrive first at its end of stroke and to activate flow switch 445 in the kidney/spent circuit as shown at instant 649 prior to actuation of inlet flow switch 455 at instant 650. In the interim between instants 649 and 650, for example, 1 to 2 seconds, pump current rises sharply as shown at 651 and relay of this steep rise to the Diaphragm Synchronization Control 476 from Current Monitor Interface 532 causes a preprogrammed electrical signal from control 476 through Motor Driver 534 to drop the Pump Motor voltage to zero as shown at 652. At time instant 650 when the valves switch to valve state A the normal voltage is restored to pump 530 as shown at 653.

## Claims

1. A closed hydraulic circuit comprising a hemodialyzer (510) separated by a semipermeable membrane into a blood side and a dialysate side and having an inlet port (527) and an autlet port (529), and arranged such that the operator presetting of the rate of withdrawal of ultrafiltrate from said circuit induces automatically those operating parameters in the circuit which produce water removal from the blood so as to insure maintainence of the rate of water separation across the semipermeable membrane of the hemodialyzer to enable continuous withdrawal of ultrafiltrate at the preset withdrawal rate, a first and second cylinder unit (410, 420), each comprising a cylinder and hydraulically driven means (407, 407A) separating the associated cylinder into two chambers (401, 402) as said means moves between the ends of said cylinder, switching means in said hydraulic circuit (437, 435, 432) for operatively connecting said units with selected components of said circuit and with a drain and fresh dialysate supply source (450) on an alternating time basis such that one chamber in said first cylinder unit fills with fresh dialysate as spent dialysate is expelled from the other chamber in said first unit to said drain, and during that same time interval one chamber in said second cylinder unit fills with spent dialysate from said outlet port of said hemodialyzer as fresh dialysate is expelled from the other chamber in said second unit to said inlet port of said hemodialyzer, and said functions alternate after simultaneous switching occurs, said selected components of said circuit comprising supply line means (460) for alternately connecting said cylinder units with said inlet port of said hemodialyzer and also comprising spent dialysate line means (470, 440) for alternately connecting said cylinder units with said outlet port of said hemodialyzer, means (445, 455) for sensing the end of stroke of each said hydraulically driven means in each of said first and second cylinder units, means (478), for operating said switching means to alternate said functions of said first and second cylinder units, characterised in that said fresh dialysate supply is bubble free, said means (478) for operating said switching means is responsive to the instant of latest arrival of the end of stroke of said hydraulically driven means in said first and second cylinder units, and in that there are provided means (440) for removing gas bubbles from said spent dialysate before said spent dialysate enters a chamber in either of said first and said second cylinder units, presettable means (430) in said circuit for withdrawing bubble- free dialysate therefrom at a predetermined rate, means (476) for detecting the time difference between the instant of each end of stroke of each of said hydraulically driven means and for converting said detected time difference into a flow rate electrical signal, and dialysate flow control means (530) located in said spent dialysate line (470) and responsive to said flow rate signal for controlling the spent dialysate flow rate in said spent dialysate line (470) to reduce said time difference upon continued operation of said circuit.

2. A circuit as claimed in claim 1, having means for sensing and converting pressure changes in said spent dialysate removal line upstream of said dialysate flow control means (530) and for converting said pressure changes into electrical signals to said dialysate flow control means on a repetitive, continuous basis for re-establishing the rate of dialysate flow through said dialysate flow control means at the instant prior to said sensed change in said pressure.

3. A circuit as claimed in claim 1 or 2, comprising means for supplying said flow rate signal to said control means (530) at the commencement of the next alternate half cycle of movement of said hydraulically driven means in the same direction that provided the detected time difference from which said flow rate signal was converted.

4. A circuit as claimed in claim 1, 2 or 3, said presettable means (430) being connected with one of said selected component (460, 470) of said circuit for withdrawing said bubble-free dialysate therefrom.

5. A circuit as claimed in any one of claims 1 to 4, comprising pressure reducing means (520) for maintaining a reduced pressure at said kidney inlet port with respect to the pressure at said fresh dialysate supply source (450).

6. A closed hydraulic circuit according to claim 1, comprising an automated single pass negative pressure dialysate hemodialysis treatment circuit, said first cylinder unit (410) comprising a first cylinder and first hydraulically driven separating means (407) separating said first cylinder into two chambers (401, 402), said first separating means being movable between the ends of said first cylinder, said first cylinder unit being alternately connected to a fresh/drain circuit for filling one of the associated chambers via a dialysate supply line (435) with fresh dialysate as spent dialysate is removed from the other of said associated chambers to drain via a drain line (480) and being alternately connected to a kidney/spent circuit for filling the other of said chambers with spent dialysate via a kidney spent dialysate removal line (470) as the fresh dialysate is removed from the one of said chambers and supplied to the inlet port (527) of said hemodialyzer via kidney dialysate supply line (460);

said second cylinder unit (420) comprising a second cylinder and second hydraulically driven separating means (407A) operable independently of said first hydraulically driven separating means and separating said second cylinder into two chambers (401A, 402A) said second separating means being movable between the ends of said second cylinder, said second cylinder unit being connected to said kidney/spent circuit during the time said first cylinder unit is connected to said fresh/drain circuit and being alternately connected to said fresh/drain circuit during the time said first cylinder unit is connected to said kidney/ spent circuit;

said switching means comprising valve switching means (432, 435, 437, 439) associated with said first and said second cylinders for connecting said cylinders alternately with one of said fresh/drain and said kidney/spent circuits;

said dialysate flow control means (530) being connected into said kidney spent dialysate removal line (470) intermediate said hemodialyzer outlet port (529) and said valve switching means for controlling the rate of spent dialysate flow in said kidney spent dialysate removal line (470), said circuit having pressure reducing means (520) connected into said dialysate supply line (460) intermediate said kidney dialysate inlet port and said valve switching means, said dialysate flow control means (530) and pressure reducing means (520) preserving an above atmospheric circuit portion (400) in said spent dialysate removal line and dialysate supply line, respectively, downstream of said dialysate flow control means and upstream of said pressure reducing means, said spent dialysate line means comprising said kidney spent dialysate removal line (470) and a degasifier (440) connected into said kidney spent dialysate removal line intermediate said dialysate flow control means (530) and said valve switching means in said kidney/spent circuit; said presettable means (430) being connected into said above atmospheric circuit portion (400), said means (476) for converting said detected time difference into said flow rate electrical signal comprising flow rate signal generator means for supplying to said dialysate flow control means (530) said flow rate signal at the delayed instant of the end of the succeeding half cycle of traverse to the same end of stroke from which the said detected time difference first occurred for causing said dialysate flow control means (530) to adjust the rate of dialysate flow in said kidney spent dialysate removal line and thereby to achieve synchronization of the instant of end of stroke of each of said hydraulically driven means in said cylinders.

7. An automated hemodialysis treatment circuit as claimed in claim 6, wherein said hemodialyzer is a hollow fiber artificial kidney.

8. An automated hemodialysis treatment circuit as claimed in claim 6 or 7 wherein said circuit includes pressure sensing means (490) in said spent dialysate removal line (470) intermediate said dialysate flow control means (530) and said hemodialyzer outlet port (529) for sensing pressure changes in spent dialysate in said spent dialysate removal line (470) and for converting said pressure change into an electrical signal for supply to said dialysate flow control means (530) to re-establish the rate of dialysate flow through said dialysate flow control means (530) that existed immediately prior to said detected change in said pressure.

9. An automated hemodialysis treatment circuit as claimed in claim 6 or 7 wherein said circuit includes a first sensing means (455) consisting of a flow switch intermediate said fresh dialysate source and said valve switching means in said fresh dialysate supply line, and a second sensing means (445) consisting of a flow switch in said kidney/spent circuit intermediate said dialysate flow control means and said valve switching means in said spent dialysate removal line.

10. An automated hemodialysis treatment circuit as claimed in claim 9 wherein said circuit includes pump means (458) in said fresh/drain circuit intermediate said fresh dialysate source and upstream of said valve switching means in said fresh dialysate supply line.

11. An automated hemodialysis treatment circuit as claimed in any one of claims 6 to 9 wherein said dialysate flow control means includes a pump (530).

12. An automated hemodialysis treatment circuit as claimed in any one of claims 6 to 11 wherein said hydraulically driven means is a diaphragm (407, 407A).

**Revendications**

1. Circuit hydraulique fermé comprenant un hémodialyseur (510) subdivisé par une membrane semi-perméable en un côté sang et un côté liquide de dialyse et doté d'un orifice (527) d'entrée et d'un orifice (529) de sortie et monté de façon telle que le préréglage par un opérateur du débit de soutirage de l'ultrafiltrat provenant de ce circuit induit automatiquement dans le circuit les paramètres de fonctionnement qui produisent l'élimination d'eau du sang, avec pour effet d'assurer la constance du débit de séparation de l'eau à travers la membrane semi-perméable de l'hémodialyseur, dans le but de permettre un soutirage continu de l'ultrafiltrat, au débit préréglé de soutirage, un premier et un second éléments cylindriques (410, 420), chacun de ceux-ci comprenant un cylindre et des moyens (407, 407A) commandés hydrauliquement et subdivisant le cylindre associé en deux chambres (401, 402) lorsque ces moyens se déplacent entre les extrémités du cylindre, des moyens de manoeuvre dans ce circuit hydraulique (437, 435, 432) pour relier opérationnellement les éléments cylindrique avec les éléments sélectionnés du circuit et avec une évacuation et une source (450) d'alimentation en liquide frais de dialyse, suivant un régime alterné tel qu'une chambre du premier élément cylindrique se remplit de liquide frais de dialyse tandis que du liquide épuisé de dialyse est expulsé de l'autre chambre de ce premier élément cylindrique vers l'évacuation et que pendant le même laps de temps, une chambre du second élément cylindrique se remplit de liquide épuisé de dialyse provenant de l'orifice de sortie de l'hémodialyseur tandis que du liquide frais de dialyse est expulsé de l'autre chambre du second élément cylindrique vers l'orifice d'entrée de l'hémodialyseur, et ces fonctions alternent après que se produit une maneuvre simultanée de robinets, les éléments sélectionnés du circuit comprenant un moyen (460) formant une conduite d'alimentation pour relier alternativement les éléments cylindriques à l'orifice d'entrée de l'hémodialyseur, et comprenant aussi des moyens (470, 440) formant une conduite de liquide épuisé de dialyse pour relier alternativement les éléments cylindriques à l'orifice de sortie de l'hémodialyseur, des moyens (445, 455) pour détecter la fin de course de chacun des moyens commandés hydrauliquement dans chacun des premier et second éléments cylindriques, un moyen (478) pour commander le moyen de maneuvre des robinets afin de réaliser l'alternance des fonctions des premier et second éléments cylindriques, caractérisé en ce que l'alimentation en liquide frais de dialyse est exempt de bulles, le moyen (478) pour commander le moyen de maneuvre réagit à l'instant de la dernière arrivée en fin de course des moyens commandés hydrauliquement dans les premier et second éléments cylindriques et ce que l'on a prévu un moyen (440) pour éliminer les bulles de gaz du liquide épuisé de dialyse avant que ce liquide épuisé de dialyse ne pénétre dans une chambre dans l'un ou l'autre des premier et second éléments cylindriques, un moyen préréglable (430) dans ledit circuit pour soutirer de ce circuit le liquide de dialyse exempt de bulles à un débit prédéterminé, un moyen (476) pour détecter l'écart de temps entre le moment de chaque fin de course de chacun des moyens commandés hydrauliquement et pour convertir cet écart de temps détecté en un signal électrique de débit, un moyen (530) de régulation du débit du liquide de dialyse logé dans la conduite (470) de liquide épuisé de dialyse et sensible à ce signal de débit pour réguler le débit de liquide épuisé de dialyse dans la conduite (470) de liquide épuisé de dialyse afin de réduire cet écart de temps en fonctionnement continu du circuit.

2. Circuit suivant la revendication 1, comprenant un moyen pour détecter et convertir les variations de pression dans la conduite d'évacuation du liquide épuisé de dialyse en amont du moyen (530) de régulation du débit de liquide de dialyse et pour convertir ces variations de pression en signaux électriques envoyés au moyen de régulation du flux de liquide de dialyse suivant un rythme répétitif et continu, dans le but de ramener le débit du liquide de dialyse, par l'intermédiaire du moyen de régulation du débit du liquide de dialyse, à la valeur qui était la sienne à l'instant précédant cette variation détectée de pression.

3. Circuit suivant l'une des revendications 1 ou 2, comprenant un moyen pour envoyer le signal de débit au moyen (530) de régulation au commencement du demi-cycle suivant alterné de déplacement du moyen commandé hydrauliquement dans le même sens que celui qui avait provoqué l'écart de temps détecté à partir duquel le signal de débit avait été converti.

4. Circuit suivant l'une quelconque des revendications 1, 2 ou 3, dans lequel le moyen (430) préréglable est relié à l'un des éléments sélectionnés (460, 470) du circuit pour soutirer de ce circuit le liquide de dialyse exempt de bulles.

5. Circuit suivant l'une quelconque des revendications 1, à 4, comprenant un moyen (520) pour réduire la pression afin de maintenir une pression réduite à l'orifice d'entrée du rein par rapport à la pression à la source (450) d'alimentation en liquidé frais de dialyse.

6. Circuit hydraulique fermé suivant la revendication 1, comprenant un circuit de traitement automatisé par hémodialyse à un seul passage et à pression negative du liquide de dialyse, dans lequel le premier élément cylindrique (410) comprend un premier cylindre et un premier moyen (407) de séparation à commande hydraulique subdivisant ce premier cylindre en deux chambres (401, 402), ce premier moyen de séparation étant mobile entre les extrémités du premier cylindre, le premier élément cylindrique étant relié alternativement à un circuit liquide frais - évacuation pour remplir de liquide frais de dialyse l'une des chambres associées par l'intermédiaire d'une conduite (435) d'alimentation en liquide de dialyse, en même temps que du liquide épuisé de dialyse est expulsé de l'autre chambre associée et évacué par une conduite (480) d'évacuation, et étant relié alternativement à un circuit rein - liquide épuisé pour remplir l'autre chambre avec du liquide épuisé de dialyse par l'intermédiaire d'une conduite (470) d'évacuation de liquide épuisé de dialyse du rein, en même temps que le liquide frais de dialyse est expulsé de l'une des chambres et envoyé à l'orifice (527) d'entrée de l'hémodialyseur par l'intermédiaire d'une conduite (460) d'alimentation rein - liquide de dialyse; le second élément cylindrique (420) comprend un second cylindre et un second moyen (407A) de séparation à commande hydraulique manoeuvrable indépendamment du premier moyen de séparation à commande hydraulique et subdivisant ce second cylindre en deux chambres (401a, 402A), ce second moyen séparateur étant mobile entre les extrémités du second cylindre, le second élément cylindrique étant relié au circuit rein - liquide épuisé pendant que le premier élément cylindrique est relié au circuit liquide frais - évacuation et étant relié alternativement au circuit liquide frais - évacuation pendant que le premier élément cylindrique est relié au circuit rein - liquide épuisé; le moyen de manoeuvre comprend des moyens (432, 435, 437, 439) de manoeuvre des robinets associés au premier et au second cylindres pour relier ces cylindres alternativement à l'un des circuits liquide frais - évacuation et rein - liquide épuisé; le moyen (530) de régulation du débit de liquide de dialyse est relié à la conduite (470) d'évacuation du liquide épuisé de dialyse du rein situé entre l'orifice (529) de sortie de l'hémodialyseur et le moyen de manoeuvre des robinets pour assurer la régulation du débit du liquide épuisé de dialyse dans la conduite (470) d'évacuation rein - liquide épuisé de dialyse, ce circuit disposant de moyens (520) de réduction de la pression reliés à la conduite (460) d'alimentation en liquide de dialyse du rein et le moyen de manoeuvre des robinets, le moyen (530) de régulation du débit du liquide de dialyse et le moyen (520) de réduction de la pression maintenant une section (400) de circuit où la pression est supérieure à la pression atmosphérique dans la conduite d'évacuation du liquide épuisé de dialyse et dans la conduite d'alimentation en liquide de dialyse, respectivement, en aval du moyen de régulation du débit du liquide de dialyse et en amont du moyen de réduction de la pression, le moyen formant conduite du liquide épuisé de dialyse du rein comprenant la conduite (470) d'évacuation du liquide épuisé de dialyse du rein et un dégazeur (440) branché sur la conduite d'évacuation du liquide épuisé de dialyse du rein entre le moyen (530) de régulation du débit du liquide de dialyse et le moyen de manoeuvre des robinets dans le circuit rein - liquide épuisé, le moyen préréglable (430) étant branché sur la section (400) à pression supérieure à la pression atmosphérique, le moyen (476) pour convertir l'écart détecté de temps en signal électrique de débit comprenant un moyen générateur de signaux de débit pour envoyer au moyen (530) de régulation du débit de liquide de dialyse le signal de débit à l'instant retardé de la fin du demi-cycle suivant de déplacement transversal vers la même fin de course à partir de laquelle s'est d'abord produit cet écart détecté de temps afin que le moyen (530) de régulation du débit du liquide de dialyse règle le débit du liquide de dialyse dans la conduite d'évacuation rein - liquide épuisé de dialyse et, de ce fait, réalise le synchronisme du moment de fin de course de chacun des moyens commandés hydrauliquement dans les cylindres.

7. Circuit de traitement automatisé par hémodialyse suivant la revendication 6, dans lequel l'hémodialyseur est un rein artificiel à fibres creuses.

8. Circuit de traitement automatisé par hémodialyse suivant l'une des revendications 6 ou 7, dans lequel ce circuit comprend un moyen (490) de détection de pression dans la conduite (470) d'évacuation du liquide épuisé de dialyse, placé entre le moyen (530) de régulation du débit du liquide de dialyse et l'orifice (529) de sortie de l'hémodialyseur, pour détecter les variations de pression du liquide épuisé de dialyse dans la conduite (470) d'évacuation du liquide épuisé de dialyse et pour convertir cette variation de pression en un signal électrique envoyé au moyen (530) de régulation du débit du liquide de dialyse pour rétablir, par l'intermédiaire de ce moyen (530) de régulation du débit de liquide de dialyse, le débit du liquide de dialyse qui existait immédiatement avant cette variation détectée de pression.

9. Circuit de traitement automatisé par hémodialyse suivant l'une des revendications 6 ou 7, dans lequel ce circuit comprend un premier moyen (455) de détection consistant en un robinet de réglage du débit placé entre la source de liquide frais de dialyse et le moyen de manoeuvre des robinets dans la conduite d'alimentation en liquide frais de dialyse, et un second moyen (445) de détection consistant en un robinet de réglage du débit dans le circuit rein - liquide épuisé, placé entre le moyen de régulation du débit de liquide de dialyse et le moyen de manoeuvre des robinets dans la conduite d'évacuation du liquide épuisé de dialyse.

10. Circuit de traitement automatisé par hémodialyse suivant la revendication 9, dans lequel ce circuit comprend un moyen (458) de pompage dans le circuit liquide frais - évacuation, placé entre la source de liquide frais de dialyse et en amont du moyen de manoeuvre des robinets dans la conduite d'alimentation en liquide frais de dialyse.

11. Circuit de traitement automatisé par hémodialyse, suivant l'une quelconque des revendications 6 à 9, dans lequel le moyen de régulation du débit de liquide de dialyse comprend une pompe (530).

12. Circuit de traitement automatisé par hémodialyse, suivant l'une quelconque des revendications 6 à 11, dans lequel le moyen commandé hydrauliquement est une membrane (407, 407A).

**Patentansprüche**

1. Geschlossener Hydraulikkreis mit einem Hämodialysator (510), der von einer semipermeablen Membran in eine Blutseite und eine Dialyseseite geteilt ist und eine Einlaßöffnung (527) und eine Auslaßöffnung (529) aufweist und derart angeordnet ist, daß die Operateur-Vorgabe der Entnahmerate des Ultrafiltrats aus dem Kreis automatisch diejenigen Betriebsparameter im Kreis induziert, die den Wasserentzug aus dem Blut hervorrufen, so daß die Aufrechterhaltung der Wasserscheiderate durch die semipermeable Membran des Hämodialysators gewährleistet und eine kontinuierliche Entnahme des Ultrafiltrats mit der vorgegebenen Entnahmerate ermöglicht wird, einer ersten und einer zweiten Zylindereinheit (410, 420) mit jeweils einem Zylinder und einer hydraulisch betriebenen Einrichtung (407, 407A), die den jeweiligen Zylinder in zwei Kammern (401, 402) teilt und sich zwischen den Enden des Zylinders bewegt, einer Schalteinrichtung im Hydraulikkreis (437, 435, 432) zum betriebsfähigen Verbinden der Einheiten mit ausgewählten Komponenten des Kreises und mit einem Abfluß und einer Vorratsquelle (450) für frisches Dialysat auf einer alternierenden Zeitbasis, so daß eine Kammer in der ersten Zylindereinheit sich mit frischem Dialysat füllt, wenn verbrauchtes Dialysat aus der anderen Kammer in der ersten Einheit zum Abfluß ausgestoßen wird, und während des gleichen Zeitintervalles eine Kammer in der zweiten Zylindereinheit sich mit verbrauchtem Dialysat aus der Auslaßöffnung des Hämodialysators füllt, wenn frisches Dialysat aus der anderen Kammer in der zweiten Einheit zur Einlaßöffnung des Hämodialysators ausgestoßen wird, und diese Funktionen nach einem simultanen Schaltvorgang umgeschaltet werden, wobei die ausgewählten Komponenten des Kreises eine Versorgungsleitung (460) zum abwechselnden Verbinden der Zylindereinheiten mit der Einlaßöffnung des Hämodialysators aufweisen und jeweils Leitungen (470, 440) für das verbrauchte Dialysat zum abwechselnden Verbinden der Zylindereinheiten mit der Auslaßöffnung des Hämodialysators einer Einrichtung (445, 455) zum Abtasten des Hubendes jeder hydraulisch betriebenen Einrichtung in der ersten und in der zweiten Zylindereinheit, einer Einrichtung (478) zum Betätigen der Schalteinrichtung zum Umschalten der Funktionen der ersten und der zweiten Zylindereinheit, dadurch gekennzeichnet, daß der Vorrat an frischem Dialysat blasenfrei ist, daß die Einrichtung (478) zum Betätigen der Schalteinrichtung auf den Augenblick des spätesten Auftretens des Hubendes der hydraulisch betriebenen Einrichtungen in der ersten und der zweiten Zylindereinheit anspricht, und ferner durch eine Einrichtung (440) zum Entfernen von Gasblasen aus dem verbrauchten Dialysat, bevor das verbrauchte Dialysat in eine Kammer in der ersten oder der zweiten Zylindereinheit eintritt, eine Vorgabeeinheit (430) im Kreis zum Entnehmen von blasenfreiem Dialysat aus dem Kreis mit einer vorgegebenen Rate einer Einrichtung (476) zum Ermitteln der Zeitdifferenz zwischen dem Augenblick jedes Hubendes jeder hydraulisch betriebenen Einrichtung und zum Umwandeln der ermittelten Zeitdifferenz in ein die Strömungsrate anzeigendes elektrisches Signal, und einer Strömungskontrolleinrichtung (530) für das Dialysat, die in der Leitung (470) für das verbrauchte Dialysat angeordnet ist und auf das Strömungsratensignal anspricht, um die Strömungsrate des verbrauchten Dialysats in der Leitung (470) für das verbrauchte Dialysat zu kontrollieren und die Zeitdifferenz beim weiteren Betrieb des Kreises zu verringern.

2. Kreis nach Anspruch 1, mit einer Einrichtung zum Abtasten und Umwandeln von Druckänderungen in der Entzugsleitung für das verbrauchte Dialysat stromaufwärts von der Dialysat-Strömungskontrolleinrichtung (530) und zum wiederholten, kontinuierlichen Umwandeln der Druckänderungen in elektrische Signale an die Dialysat-Strömungskontrolleinrichtung, um mittels der Dialysat-Strömungskontrolleinrichtung die Dialysat-Strömungsrate im Augenblick vor der abgetasteten Druckänderung wiederherzustellen.

3. Kreis nach Anspruch 1 oder 2, gekennzeichnet durch eine Einrichtung zum Zuführen des Strömungsratensignals an die Kontrolleinrichtung (530) am Beginn des nächsten alternierenden halben Bewegungszykluses der hydraulisch betriebenen Einrichtung in der gleichen Richtung, die die ermittelte Zeitdifferenz lieferte, aus der das Strömungsratensignal umgewandelt wurde.

4. Kreis nach Anspruch 1, 2 oder 3, wobei die Vorgabeeinrichtung (430) mit einer der auswählbaren Komponenten (460, 470) des Kreises verbunden ist, um das blasenfreie Dialysat daraus zu entnehmen.

5. Kreis nach einem der Ansprüche 1 bis 4, gekennzeichnet durch eine Druckreduziereinrichtung (520) zum Aufrechterhalten eines reduzierten Druckes an der Nieren Einlaßöffnung bezüglich des Druckes an der Vorratsquelle (450) für frisches Dialysat.

6. Geschlossener Hydraulikkreis nach Anspruch 1, mit einem automatischen, einstufigen Unterdruck-Dialysat-Hämodialyse-Behandlungskreis, wobei die erste Zylindereinheit (410) einen ersten Zylinder und eine erste, hydraulisch betriebene Trenneinrichtung (407) aufweist, die den ersten Zylinder in zwei Kammern (401, 402) unterteilt, wobei die erste Trenneinrichtung zwischen den Enden des ersten Zylinders beweglich ist und die erste Zylindereinheit alternierend mit einem Frischdialysat/Abfluß-Kreis zum Füllen einer der jeweiligen Kammern mit frischem Dialysat über die Dialysat-Versorgungsleitung (435) verbunden ist, wenn verbrauchtes Dialysat aus der anderen der jeweiligen Kammern entzogen und über eine Abflußleitung (480) abgelassen wird, und alternierend mit einem Niere/Verbrauchtdialysat-Kreis zum Füllen der anderen der Kammern mit verbrauchtem Dialysat über eine Entzugsleitung (470) für von der Niere verbrauchtes Dialysat verbunden ist, wenn das frische Dialysat von der ersten Kammer entzogen und über eine Nieren-Dialysat-Versorgungsleitung (460) der Einlaßöffnung (527) des Hämodialysators zugeführt wird; wobei die zweite Zylindereinheit (420) einen zweiten Zylinder und eine zweite hydraulisch betriebene Trenneinrichtung (407A) aufweist, die unabhängig von der ersten, hydraulisch betriebenen Trenneinrichtung betreibbar ist und den zweiten Zylinder in zwei Kammern (401A, 402A) unterteilt, wobei die zweite Trenneinrichtung zwischen den Enden des zweiten Zylinders beweglich ist und die zweite Zylindereinheit mit dem Niere/Verbrauchtdialysat-Kreis während der Zeit verbunden ist, in der der erste Zylinder mit dem Frischdialysat/Abfluß-Kreis verbunden ist, und alternierend mit dem Frischdialysat/Abfluß-Kreis während der Zeit verbunden ist, in der die erste Zylindereinheit mit dem Niere/Verbrauchtdialysat-Kreis verbunden ist, wobei die Schalteinrichtung eine mit dem ersten und dem zweiten Zylinder verknüpfte Ventilschalteinrichtung (432, 435, 437, 439) zum alternierenden Verbinden der Zylinder mit dem Frischdialysat/Abfluß-Kreis oder dem Niere/Verbrauchtdialysat-Kreis aufweist, wobei die Dialysat-Strömungskontrolleinrichtung (530) in die Entzugsleitung (470) für von der Niere verbrauchtes Dialysat zwischen der Hämodialysator-Auslaßöffnung (529) und der Ventilschalteinrichtung zwischengeschaltet ist, um die Strömungsrate des verbrauchten Dialysats in der Entzugsleitung (450) für von der Niere verbrauchtes Dialysat zu kontrollieren, wobei der Kreis eine in die Dialysat-Versorgungsleitung (460) zwischen die Niere-Dialysat-Einlaßöffnung und die Ventilschalteinrichtung zwischengeschaltete Druckreduziereinrichtung aufweist, wobei die Dialysat-Strömungskontrolleinrichtung (530) und die Druckreduziereinrichtung (520) in der Entzugsleitung für verbrauchtes Dialysat bzw. in der Dialysat-Versorgungsleitung einen über Atmosphärendruck befindlichen Abschnitt (400) des Kreises aufrechterhalten, stromabwärts von der Dialysat-Strömungskontrolleinrichtung und stromaufwärts von der Druckreduziereinrichtung,

wobei die Leitung für verbrauchtes Dialysat die Entzugsleitung (470) für von der Niere verbrauchtes Dialysat und einen zwischen der Dialysat-Strömungskontrolleinrichtung (530) und der Ventilschalteinrichtung im Niere/Verbrauchtdialysate-Kreis in die Entzugsleitung des von der Niere verbrauchten Dialysats zwischengeschalteten Entgaser (440) aufweist, wobei die Vorgabeeinrichtung (430) in den oberhalb Atmosphärendruck befindlichen Abschnitt (400) des Kreises geschaltet ist, wobei die Einrichtung (476) zum Umwandeln der ermittelten Zeitdifferenz in das die Strömungsrate anzeigende elektrische Signal eine Strömungsraten-Signalgeneratoreinrichtung aufweist zum Zuführen des Strömungsratensignales an die Dialysat-Strömungskontrolleinrichtung (530) im verzögerten Augenblick des Endes des darauffolgenden halben Bewegungszyklusses zum gleichen Hubende, von dem die ermittelte Zeitdifferenz zuerst auftrat, wodurch die Dialysat-Strömungskontrolleinrichtung (530) die Strömungsrate des Dialysats in der Entzugsleitung des von der Niere verbrauchten Dialysats einstellt und dadurch eine Synchronisation des Augenblickes des Hubendes jeder der hydraulisch betriebenen Einrichtungen in den Zylindern erzielt.

7. Automatischer Hämodialyse-Behandlungskreis nach Anspruch 6, dadurch gekennzeichnet, daß der Hämodialysator eine künstliche Niere aus Hohlfaser ist.

8. Automatischer Hämodialyse-Behandlungskreis nach Anspruch 6 oder 7, dadurch gekennzeichnet, daß der Kreis eine Druckabtasteinrichtung (490) in der Entzugsleitung (470) für das verbrauchte Dialysat zwischen der Dialysat-Strömungskontrolleinrichtung (530) und der Hämodialysator-Auslaßöffnung (529) aufweist zum Abtasten von Druckänderungen im verbrauchten Dialysat in der Entzugsleitung (470) für das verbrauchte Dialysat und zum Umwandeln der Druckänderung in ein der Dialysat-Strömungskontrolleinrichtung (530) zuzuführendes elektrisches Signal, um die Dialysat-Strömungsrate durch die Dialysat-Strömungskontrolleinrichtung (530) wiederherzustellen, die unmittelbar vor der ermittelten Druckänderung bestand.

9. Automatischer Hämodialyse-Behandlungskreis nach Anspruch 6 oder 7, dadurch gekennzeichnet, daß der Kreis eine aus einem zwischen der Quelle für frisches Dialysat und der Ventilschalteinrichtung in der Versorgungsleitung für frisches Dialysat angeordneten Strömungsschalter bestehende erste Abtasteinrichtung (455) und eine aus einem in dem Niere/Verbrauchtdialysat-Kreis zwischen der Dialysat-Strömungskontrolleinrichtung und der Ventilschalteinrichtung in der Entzugsleitung für verbrauchtes Dialysat angeordneten Strömungsschalter bestehende zweite Abtasteinrichtung (445) aufweist.

10. Automatischer Hämodialyse-Behandlungskreis nach Anspruch 9, dadurch gekennzeichnet, daß der Kreis eine im Frischdialysat/Abfluß-Kreis zwischen der Quelle für frisches Dialysat und stromaufwärts von der Ventilschalteinrichtung in der Versorgungsleitung für frisches Dialysat angeordnete Pumpeinrichtung (458) aufweist.

11. Automatischer Hämodialyse-Behandlungskreis nach einem der Ansprüche 6 bis 9, dadurch gekennzeichnet daß die Dialysat-Strömungskontrolleinrichtung eine Pumpe (530) aufweist.

12. Automatischer Hämodialyse-Behandlungskreis nach einem der Ansprüche 6 bis 11, dadurch gekennzeichnet, daß die hydraulisch betriebene Einrichtung ein Diaphragma (407, 407A) ist.

FIG. 1

FIG. 2

PATIENT

0 033 222

FIG. 3

DIAPHRAGM CONTROL VALVES

432
439
435
431
455

INLET FLOW SWITCH

FLOW SWITCH INTERFACE — 479

KIDNEY LOOP PUMP — 530

METERING PUMP — 430

490

DIALYSATE PRESSURE TRANSDUCER

552

TACH

KIDNEY FLOW SWITCH — 445

CURRENT MONITOR INTERFACE — 532

FLOW SWITCH INTERFACE — 477

536

TRANSDUCER SIGNAL CONDITIONING

554

TACHOMETER SIGNAL CONDITIONING

SOLENOID DRIVER — 478

MOTOR DRIVER — 534

560

MOTOR DRIVER

DIAPHRAGM SYNCHRONIZATION & VALVE SWITCHING CONTROL — 476

SPEED CONTROL — 556

UFR CONTROL

MICROPROCESSOR CONTROL UNIT — 480

DESIRED UFR SET POINT

0 033 222

FIG. 4

FIG. 5

FIG. 6

FIG. 7

FIG. 8

11

FIG. 9

FIG. 10

0 033 222

FIG. 11

FIG. 12

15

# FIG. 13

FLOW SWITCH "INLET" — ON / OFF

FLOW SWITCH "KIDNEY" — ON / OFF

VALVE STATE — A / B

PUMP MOTOR VOLTAGE — MAX / O — 647

DIALYSATE PRESSURE — O / -700mmHg — 629 — 648

TIME ⟶

# FIG. 14

FLOW SWITCH "INLET" — ON / OFF — 649 — 650

FLOW SWITCH "KIDNEY" — ON / OFF

VALVE STATE — A / B

PUMP MOTOR VOLTAGE — MAX / O — 655 — 652

DIALYSATE PRESSURE — O / -700 mmHg

PUMP MOTOR CURRENT — MAX / O — 651

TIME ⟶